# EUROPEAN PATENT SPECIFICATION

(11) **EP 3 755 213 B1**
(45) Date of publication and mention of the grant of the patent: **13.09.2023**
(21) Application number: 19874774.3
(22) Date of filing: 30.04.2019
(51) Int. Cl.: A61K 35/12, A61K 35/34, A61K 35/54, A61P 21/00, C12N 5/00, A61B 5/00

(54) **USES OF FETAL TISSUE EXTRACT**
VERWENDUNGEN VON FÖTALGEWEBEEXTRAKT
UTILISATIONS D' UN EXTRAIT DE TISSUS FOETAUX

(43) Date of publication of application: 30.12.2020
(73) Proprietor: Alis Pharma Ltd., Central, Hong Kong 999077 (CN)
(72) Inventor: LUI, May Pui-Man, Hong Kong 999077 (CN)
(74) Representative: Rüger Abel Patentanwälte PartGmbB
(86) International application number: PCT/CN2019/085202
(87) International publication number: WO 2020/220265

(56) References cited:
- WO-A1-2014/039654
- WO-A1-2018/128779
- WO-A1-2019/080942
- CN-A- 1 135 890
- CN-A- 105 408 472
- CN-A- 109 679 897
- US-A1- 2003 017 587
- US-A1- 2013 336 935
- US-A1- 2016 058 799
- US-A1- 2017 327 792
- NATHALIE HIRT-BURRI ET AL: "Human muscular fetal cells: a potential cell source for muscular therapies", PEDIATRIC SURGERY INTERNATIONAL, SPRINGER, BERLIN, DE, vol. 24, no. 1, 26 October 2007 (2007-10-26), pages 37-47, XP019588862, ISSN: 1437-9813
- QU-PETERSEN Z ET AL: "Identification of a novel population of muscle stem cells in mice: Potential for muscle regeneration", THE JOURNAL OF CELL BIOLOGY, THE ROCKEFELLER UNIVERSITY PRESS, US, vol. 157, no. 5, 27 May 2002 (2002-05-27), pages 851-864, XP002302343, ISSN: 0021-9525, DOI: 10.1083/JCB.200108150

## Description

### FIELD OF THE INVENTION

The present disclosure relates generally to medical products, methods for producing the same, and the uses thereof. The present disclosure relates more specifically, but not exclusively, to an extract from a fetal or newborn animal for preventing or treating muscle injury or aging associated muscle disorders or diseases.

### BACKGROUND OF THE INVENTION

The muscular system, which is the largest reservoir of protein in the body of a human being, accounts for up to 45% of the total body weight, and contributes to the maintenance of basic metabolic and functional activities. There are three kinds of muscle in a human body: skeletal muscle, cardiac muscle, and smooth muscle. Muscle disorders or diseases include but not limited to myopathy, muscle injury, fibromyalgia, chronic fatigue syndrome, polymyositis, chronic compartment syndrome, Isaac's syndrome, rhabdomyolysis, muscular dystrophy, Kennedys disease would negatively affect human health. Aging associated muscle disorders or diseases are drawing more attention in the public, particularly, in the elderly. One of the aging associated muscle disorders or diseases is sarcopenia, which is the intrinsic "myopathy" of aging.

According to the World Population Ageing 2015 reported by United Nations, the world' s population is ageing. The global aged population, aged 60 years or above will reach more than double in size by 2050, reaching an astonishing number of 2.1 billion. Ageing process is associated with numerous changes in body composition. From the age of 25 to 80, there is progressive reduction in the size and number of muscle fibers by 30% (1). Sarcopenia, firstly introduced by Irwin Rosenberg, is a prevalent ageing associated decline in muscle mass, strength and function which affects 10% of men (95% CI: 8-12%) and women (95% CI: 8-13%). Meta-analysis indicated that sarcopenia is associated with higher rate of mortality, muscle functional decline, higher rate of falls and higher incidence of hospitalization (2). Epidemiological studies indicated that muscle ageing is associated with a number of degenerative disorders (3, 4). There is a growing scientific and public interest to develop effective approaches to counteract the effects of sarcopenia in order to maintain functional independence, or "active aging."

WO 2019 / 080942 A1, which is state of the art according to Article 54(3) EPC, describes a tissue extract from a fetal or newborn animal, e.g. a sheep or rat, for preventing or treating bone disorders or diseases, the extract produced by collecting tissues or organs from the fetal or newborn animal as defined, homogenizing the collected tissues to obtain homogenates, filtrating and/or centrifuging the homogenates to obtain a supernate fluid, and purifying/processing the filtrate. The musculoskeletal tissue can originate from a newborn animal following caesarean section.

WO 2014 / 039654 A1 discloses treatment of muscle disorders such as muscular dystrophies, myopathies, and reconstitution or regeneration of muscle function in a recipient. Human fetal skeletal muscle specimens from aborted, 18-20 week gestation fetuses were prepared by two-step enzymatic digestion and gentle mechanical dissociation to first obtain bulk muscle fibers, then cells were liberated and centrifuged. Such allogeneic cells were used for treatment. Results show in comparison a reduced content of hSMPs (skeletal muscle precursor cells) in adult muscle.

WO 2018 / 128779 A1 discloses generating skeletal muscle progenitor cells (SMPCs) for use in myopathies such as Duchenne muscular dystrophy, sarcopenia, muscle wasting. The SMPCs were generated from pluripotent stem cells, such as from embryonic stem cells or induced pluripotent stem cells. Human fetal skeletal muscle weeks 8-17 were minced, dissociated, titrated, diluted and further processed.

Nathalie Hirt-Burri et al.: "Human muscular fetal cells; a potentioa cell source for muscular therapies ", PEDIATRIC SURGERY INTERNATIONAL, Springer; Berlin, Vo. 24, No. 1, 26 October 2007, pages 37-47, describes potential muscle cell therapy and tissue engineering in Duchenne Muscular Dystrophy treatment. Two human fetal skeletal muscle biopsies (15 and 16 weeks) obtained and processed in order to create a human primary fetal skeletal muscle cell bank were used as a cell source for muscle cell therapy and tissue engineering. Fetal skeletal muscle cells showed qualities required for the establishment of a cell bank. With one organ donation of 1 cm³, one could make more than 28,000 injections with 7×10⁷ cells as it was used in the clinics for human Duchenne Muscular Dystrophic patients.

Qu-Petersen Z. et al.: "Identification of a novel population of muscle stem cells in mice: Potential for muscle generation", THE JOURNAL OF CELL BIOLOGY, THE ROCKEFELLER UNIVERSITY PRESS, US, Vol. 157, No. 5, 27 May 2002, pages 851-864, discloses regeneration of functional muscle tissue in mice. MDSC (muscle-derived stem cells) from normal newborn mice were transplanted into the m. gastrocnemius of mdx mice. Primary muscle cultures were prepared from newborn (3-5 days) normal mice, skeletal muscle was minced, muscle tissue was enzymatically dissociated, and centrifuged at 3,500 rpm for 5 min. The cells were collected, incubated in dispase, and centrifuged.

US 2016 / 0 058 799 A1 discloses skeletal muscle regeneration by using minced tissue of human placentas derived following caesarean sections of male fetuses.

### SUMMARY OF THE INVENTION

It is the first time in the art to find that extract derived from fetal tissues can effectively prevent or treat aging associated muscle disorders or diseases.

Therefore, the present invention provides an extract obtained from one or more tissue(s) of a fetal or newborn animal for use in the prevention or treatment of muscle disorders or diseases in a subject, wherein the tissues are skeletal muscle, and the animal is a sheep, a goat, a pig, a rat, or a mouse, wherein the extract contains no cells, wherein the extract is produced by the steps of:
a. collecting tissues from the fetal or newborn animal;
b. homogenizing the collected tissues to obtain homogenates;
c. optionally, filtrating and/or centrifuging the homogenates to obtain a filtrate; and
d. optionally, purifying/processing the filtrate further to obtain the final products composed of fetal tissue extract.

Other aspects, features, and advantages of the methods, compositions and/or devices and/or other subject matter described herein will become apparent in the teachings set forth herein.

### DESCRIPTION OF THE FIGURES

Fig. 1 shows the effect of FSME on C2C12 cells viability by MTT assay.
Fig. 2A shows the effect of D-Gal on the viability of C2C12 cells, and Fig. 2B shows the effect of FSME on D-Gal treated C2C12 cells.
Fig. 3 shows the effect of FSME on HSMM cells viability.
Fig. 4A shows the effect of FSME on the expression of myogenic precursor markers, and Fig. 4B shows the effect of FSME on the expression of skeletal muscle markers.
Fig. 5 shows the effect of FSME on myogenic precursor markers of HSMM cells.
Fig. 6 shows the effect of FSME on muscle regeneration in C57 mice, as demonstrated via total grip strength in young C57 mice post barium chloride induced muscular injury in the tibialis anterior.
Fig. 7A shows the effect of FSME on the expression of myogenic precursor markers in C57 mice, and Fig. 7B shows the effect of FSME on the expression of skeletal muscle markers in young C57 mice post barium chloride induced muscular injury.
Fig. 8 shows the effect of FSME on muscle regeneration in C57 mice, as demonstrated via total grip strength in young C57 mice post barium chloride induced muscular injury in both the tibialis anterior and gastrocnemius.
Fig. 9 shows the effect of FSME on muscle regeneration in C57 mice, as measured by light microscopy.
Fig. 10 shows the effect of FSME on muscle regeneration in old C57 mice, as demonstrated via total grip strength test.
Fig. 11A shows the effect of FSME on the expression of myogenic precursor markers in old C57 mice, and Fig. 11B shows the effect of FSME on the expression of skeletal muscle markers in old C57 mice.

### DETAILED DESCRIPTION OF THE INVENTION

Before the present invention is described, it is to be understood that this invention is not limited to particular methods (also referred as "process" hereinafter) and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 10%.

Although any methods and materials similar or equivalent to those described herein can be used in the practice of the present disclosure, the preferred methods and materials are now described. Other embodiments will become apparent from a review of the ensuing detailed description.

In order that the invention herein described may be fully understood, the following detailed description is set forth.

The present disclosure relates to a use of a fetal tissue extract.

### A FETUS OR A NEWBORN ANIMAL

The source or donor animal of the tissues refers to the animal from which the fetal tissues are obtained. The source or donor animal is a sheep, a goat, a pig, a rat, or a mouse.

In the context of the present disclosure, the animal is a fetus or a newborn animal.

As used herein, a fetus is a stage in the prenatal development of viviparous organisms. "Fetus" refers to different timeframe for different animals. For instance, for a sheep, the fetal stage commences at the beginning of the four week post fertilization. In one embodiment, the animal is a 12-18 week fetal sheep, for instance, a 16-week fetal sheep. For instance, the "16-week fetal sheep" refers to a 16-week sheep of gestation. Preferably, the sheep is free of specific pathogen(s).

The recipient for the extract refers to the animal which receives the extract. The recipient is preferably a mammalian animal. The animal can be any animal suitable for accepting the extract or in need of receiving the extract.

In one embodiment, the recipient is a sheep, a goat, a pig, a rat, or a mouse.

In another embodiment, the recipient is a human.

In further embodiments, the recipient is allogenic or xenogenic to the donor animal. In a specific embodiment, the recipient is xenogenic to the donor animal.

### EXTRACT FROM TISSUE(S) OF A FETUS OR A NEWBORN ANIMAL

The present disclosure also relates to an extract from tissue(s) of a fetus or a newborn animal.

In a specific embodiment, the extract is a muscle extract, for instance, sheep muscle extract, more specifically, a fetal sheep muscle extract ("FSME").

"Extract," "isolate," or "tissue extract" applied interchangeably herein, refer to a complex obtained by extracting a part of a tissue or a whole tissue of a fetus or newborn animal. The commonly used solvents for extract include water or physiological saline such as PBS. In one embodiment of the present disclosure, the solvent is PBS. The extract contains no cells.

The "tissue" may include but not limited to muscle tissue or musculoskeletal tissue. Tissues from a placenta are not included in the context of the present disclosure.

In some embodiments, the tissue is muscle. Muscle is a soft tissue found in most animals, and functions to produce force and motion. They are primarily responsible for maintaining and changing posture, locomotion, as well as movement of organs.

The tissue is skeletal muscle

The extract can be produced via the following method, comprising the steps of:
a. collecting tissues from the fetal or newborn animal;
b. homogenizing the collected and cut tissues to obtain homogenates;
c. optionally, filtering and/or centrifuging the homogenates to obtain a filtrate; and
d. optionally, purifying/processing the filtrate further to obtain the final products composed of fetal tissue extract.

In one embodiment, the "filtering" in step (c) may be performed using sterile filter with desired pore size. For instance, the "filtering" in step (c) may be performed using a filter (for instance, a cell strainer) which can remove debris. For instance, the pore size of the filter is about 60-80 µm.

In one embodiment, the "purifying" in step (d) may be performed using a filter which can remove viable cells and retain bioactive components. For instance, the filter may be sterilization filter, with the pore size about <= 0.22 µm.

In one embodiment, the tissues are collected immediately from the newborn animal following caesarean section.

In a further embodiment, the homogenates are prepared using phosphate-buffered saline.

In a still further embodiment, the centrifugation is performed at 5000g for 15 min at 4 °C.

In a still further embodiment, the filtration is performed using a cell strainer. In a yet further embodiment, the extract is kept in liquid nitrogen till use.

In a specific embodiment, the method for producing the extract comprise the steps of:
a. collecting tissues from the fetal or newborn animal as defined;
b. homogenizing the collected tissues to obtain homogenates;
c. filtering via 70µm filter (also referred to hereinafter as a "cell strainer") to remove debris and/or centrifuging the homogenates to obtain a filtrate; and
d. purifying/processing the filtrate further through 0.22 µm sterilization filter to remove viable cells and bacteria to obtain the extract.

The extract of the present disclosure is effective at least in the aspects:
- promoting myoblast proliferation;
- alleviating myoblast senescence;
- promoting myogenic differentiation; and
- improving muscle strength.

### TYPICAL PROCEDURES FOR PRODUCING THE EXTRACT

A typical process for producing the extract of interest may comprise the following procedures:
- animal harvest;
- tissue collection;
- tissue processing; and
- optionally, product testing.

The procedures may subject to change/amend for optimization, which depend on the purposes of future research studies.

When the donor animal is a fetal sheep free of specific pathogens (an SPF sheep), the "animal harvest" may comprise:
- SPF pregnant sheep transport from SPF Farm to cGMP production area;
- pregnant sheep anesthesia by licensed Vet;
- sheep fetus obtained by licensed Vet by C-section in Animal Operation Theater which is sterile condition.

The "tissue collection" may comprise:
- fetus collected by operating staff, cleaned immediate and being carried to class B production area;
- dissection on sheep fetus to obtain the corresponding tissues;
- tissue washing and being carried to class A laminar flow;
- tissue cut/mesh/grind into <0.5cm³ pieces on glass petri dish placing on ice/ice pad.

The "tissue processing" may comprise:
- tissue homogenization by Glas-Col motorized homogenizer at 1600rpm for 30-120s;
- passing the homogenate through gauze filter;
- filtrate collection for cell count/viability measurement
- product formulation
- product inspection under microscope
- sample archive for future analysis.

### IMPLANT

"Implant" or "fetal tissue implant" interchangeably used herein is implanted into a zone of interest in the recipient, so as to improve the patho-physiological status of the recipient. Specifically, the implant is capable of preventing or treating muscle disorders or diseases in the recipient.

In one embodiment, the implant is an allogenic or xenogenic implant, preferably an xenogenic implant.

### PHARMACEUTICAL COMPOSITION

A pharmaceutical composition may comprise the fetal tissue extract of the present disclosure, and optionally pharmaceutically acceptable excipients.

Excipients included in the formulations are selected depending on different purposes, e.g., the mode of administration. Examples of generally used excipients included, without limitation: saline, buffered saline, dextrose, water-for-injection, glycerol, ethanol, and combinations thereof, stabilizing agents, solubilizing agents and surfactants, buffers and preservatives, tonicity agents, bulking agents, and lubricating agents.

The amount of the extract to elicit a therapeutic effect can be experimentally determined by a person skilled in the art, depending upon a variety of factors including the age, body weight, general health, sex, and diet of the subject (including, for example, whether the subject is in a fasting or fed state), the time of administration, the rate of excretion, the drug combination, and form of administration. Treatment dosages generally may be titrated to optimize safety and efficacy. Typically, dosage-effect relationships from *in vitro* and/or *in vivo* tests initially can provide useful guidance on the proper doses for subject administration.

In some embodiments, the pharmaceutical composition may be administered to a subject in accordance with known methods, such as intravenous administration, e.g., as a bolus or by continuous infusion over a period of time, by intramuscular, intraperitoneal, intracerebrospinal, subcutaneous, intra-articular, intrasynovial, intrathecal, oral, topical, or inhalation routes. In a specific embodiment, the pharmaceutical composition is administrated intramuscularly.

In another embodiment of the present disclosure, the pharmaceutical composition of the present disclosure is in the form of solid dosage forms, for example tablets (including but not limited to swallowable tablets, chewable tablets, suspension tablets, *etc.*), capsules, caplets, troches, losenges, powders, granules, *etc.*

### CONDITIONS, DISORDERS AND DISEASES

The extract from a fetal or newborn animal of the present disclosure can be used in the prevention or treatment of muscle disorders or diseases,

Muscle disorders or diseases include but not limited to myopathy, muscle injury, fibromyalgia, chronic fatigue syndrome, polymyositis, chronic compartment syndrome, Isaac's syndrome, rhabdomyolysis, muscular dystrophy, Kennedy's disease, among others.

Myopathy generally refers to multiple types of skeletal muscle diseases. One of the aging associated muscle disorders or diseases is sarcopenia, which is the intrinsic "myopathy" of aging. In some embodiment, the muscle disorder or disease is sarcopenia.

Sarcopenia is a condition characterized by loss of skeletal muscle mass and function. Although it is primarily a disease of the elderly, its development may be associated with conditions that are not exclusively seen in the elderly. Sarcopenia is a syndrome characterized by progressive and generalized loss of skeletal muscle mass and strength and it is strictly correlated with physical disability, poor quality of life and death. Risk factors for sarcopenia include age, gender and level of physical activity (5).

Muscle injury or muscle damage may involve damage to muscle, muscle fibers or attaching tendons thereof by muscle strain, pull or tear. Symptoms of a muscle injury include: (1) swelling, bruising or redness due to the injury; (2) pain at rest, (3) pain when the specific muscle or the joint in relation to that muscle is used; (4) weakness of the muscle or tendons; (5) inability to use the muscle at all. In some embodiments, the muscle injury is skeletal muscle injury. The injury may be chemical injury or physical injury. In certain embodiments, the muscle injury is chemical injury (for instance, injury induced by BaCl₂) to skeletal muscle. In one specific embodiment, the muscle injury is chemical injury (for instance, injury induced by BaCl₂) to tibialis anterior. In a further specific embodiment, the muscle injury is chemical injury (for instance, injury induced by BaCl₂) to tibialis anterior and gastrocnemius.

Myogenesis is the formation of muscular tissue, particularly, the formation of muscle fibers. Muscle fibers generally form the fusion of myoblasts into multi-nucleated fibers called myotubes. In some embodiment, the extract from a fetal or newborn animal of the present disclosure can be used in myogenesis.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed.

### Example 1: Preparation of Fetal Sheep Tissue Extract ("FSME")

- **Animal.** The fetal animal was a 16-18 gestation week fetal sheep in this example. Fetal sheep muscle was used for the preparation of FSME.
- **Collection and processing of muscle tissues.** Skeletal muscle tissues were collected immediately from the fetus following caesarean section. The muscle tissues were weighed and stored at 4°C until processing. In a preparation room, after removing the fat and connective tissues, the muscle tissues were cut into small pieces on ice bath, and then were used for preparation of FSME.
- **Preparation of FSME.** The homogenates were then prepared from the muscle tissues using phosphate-buffered saline (PBS) with homogenizer. The homogenates were filtered through sterile gauze swabs and centrifugation at 5000 g for 15 min at 4°C, followed by filtration through sterile 70µm and 0.22µm filters to remove the debris/contaminants and for sterilization. The protein content in the crude and filtered FSME was measured using BCA kit according to the manufacturer's instruction. The FSME was kept in -20°C until further use.
- **Dilution of FSME for *in vitro* and *in vivo* experiments.** For cellular experiments (*in vitro* experiments, **Examples 2-6**), sterile FSME(1) 0.5mg/ml was diluted 100 times before use to a working concentration of 5000 ng/ml. While for the animal studies (*in vivo* experiment, **Example 7**), a working concentration of 5mg/ml (100µl each limbs) was used and diluted with sterile PBS.

### Example 2: Effect of FSME on C2C12 Cell Viability

- **C2C12 cells.** Murine myoblast cell line (ATCC CRL-1772) was obtained from ATCC and used as C2C12 cells.
- **Cell culture.** The C2C12 cells were cultured in Dulbecco modified Eagle medium with high glucose supplemented with 10%(v/v) fetal bovine serum (FBS), and incubated at 37 °C in 5% CO₂. Subcultures were performed by using 0.05% trypsin solution when the confluence reached 80%.

### • MTT assay

- The cytotoxic effect of FSME on C2C12 cells was determined by 3-(4,5-dimethylthiazol-2-yl)-2,5-diphenyl tetrazolium bromide (MTT) assay. MTT works based on the oxidative phosphorylation ("oxphos") process in the cells. If the cells are alive and active, their mitochondria, the intracellular "powerhouse," is active which will provide energy to the cells via a series of oxidation and phosphorylation processes. In other words, the more cellular proliferation / viability, the darker the color, and the higher the absorbance.

Cell viability was determined by MTT assay as reflected by the activity of succinate dehydrogenase. C2C12 cells (1 × 10³ per well) were plated in 96-well microplates. Following incubation with FSME for 24 h, 48 h or 72h, 3-[4,5-dimethylthiazol-2-yl]-2,5-diphenyl tetrazolium bromide (MTT, 100 µl, 0.5 mg/ml) was added to each well and the plate was incubated at 37 °C for 3 h. The Dulbecco modified Eagle medium for cell culture was removed and replaced with 100 µl MTT Solubilization Solution, 10% Triton X-100, plus 0.1 N HCl in anhydrous isopropanol to dissolve the formazan crystals. The cell viability was quantified via spectrophotometer by measuring absorbance at 570 nm using a microplate reader.

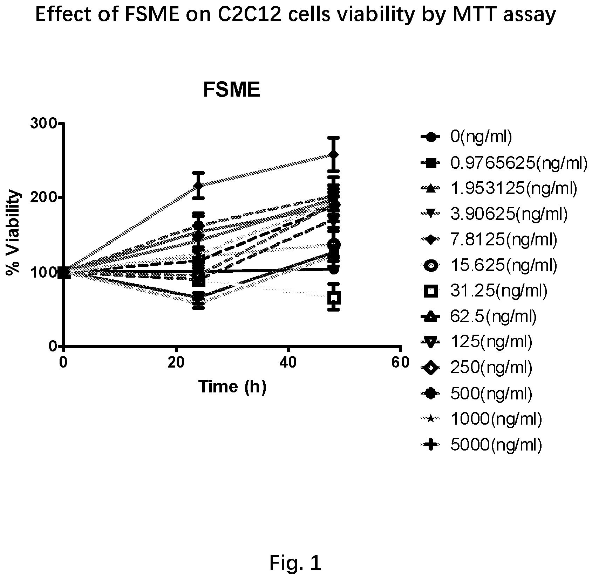

**Results:** FSME at concentrations between 0.976 ng/ mL to 1000 ng/ mL were able to promote C2C12 cell proliferation and viability (**Fig. 1**).

### Example 3: Effect of FSME on D-Gal treated C2C12 cells

Treatment of cells with D-galactose ("D-Gal") was known to produce reactive oxygen species in *in vivo* mouse and rat models and these models were used to mimic aging phenotype. In this example, C2C12 myoblast senescence model was established using D-Gal. C2C12 viability was determined by MTT assay.
- **C2C12 cells.** Murine myoblast cell line (ATCC CRL-1772) was obtained from ATCC and used as C2C12 cells.
- **Cell culture.** The C2C12 cells were cultured in Dulbecco modified Eagle medium with high glucose supplemented with 10%(v/v) fetal bovine serum (FBS), and incubated at 37 °C in 5% CO₂. Subcultures were performed by using 0.05% trypsin solution when the confluence reached 80%.

### • MTT assay

Cell viability was determined by MTT assay as reflected by the activity of succinate dehydrogenase.

***Establishment of C2C12 myoblast senescence model using D-Gal.*** C2C12 cells (1 × 10³ per well) were plated in 96-well microplates. Following incubation with D-Gal for 24 h, 48 h or 72h, 3-[4,5-dimethylthiazol-2-yl]-2,5-diphenyl tetrazolium bromide (MTT, 100 µl, 0.5 mg/ml) was added to each well and the plate was incubated at 37 °C for 3 h. The Dulbecco modified Eagle medium ("DMEM") for cell culture was removed and replaced with 100 µl MTT Solubilization Solution, 10% Triton X-100, plus 0.1 N HCl in anhydrous isopropanol to dissolve the formazan crystals. The cell viability was quantified via spectrophotometer by measuring absorbance at 570 nm using a microplate reader.

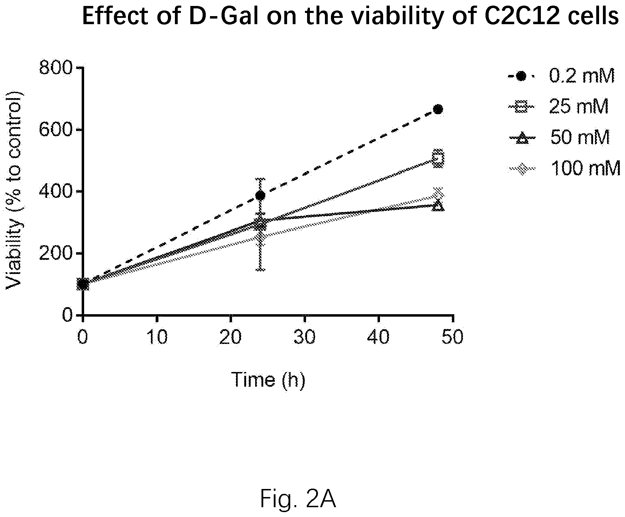

***Results:*** C2C12 myoblast senescence model was established using D-Gal. D-gal at concentrations of 25 mM, 50 mM and 100 mM was able to significantly reduce C2C12 viability (**Fig. 2A**).

***Effect of FSME on D-Gal treated C2C12 cells.*** 1000 C2C12 cells were seeded into each well of 96-well plate and were cultured in 10% (v/v) FBS supplemented high glucose DMEM in a 37°C humidified incubator with 5% CO₂. Once the cells attached, culture medium containing the specified concentration of D-gal and FSME was added to C2C12 cells. Medium was replaced every 24h with fresh medium containing D-gal and FSME. Cell viability was determined by incubating the cells with 3-(4,5-Dimethylthiazol-2-yl)-2,5-diphenyltetrazolium bromide salt solution that was added to the culture medium with cells for 3 hours until analysis by spectrophotometer with absorbance wavelengths set at 570nm and 690nm.

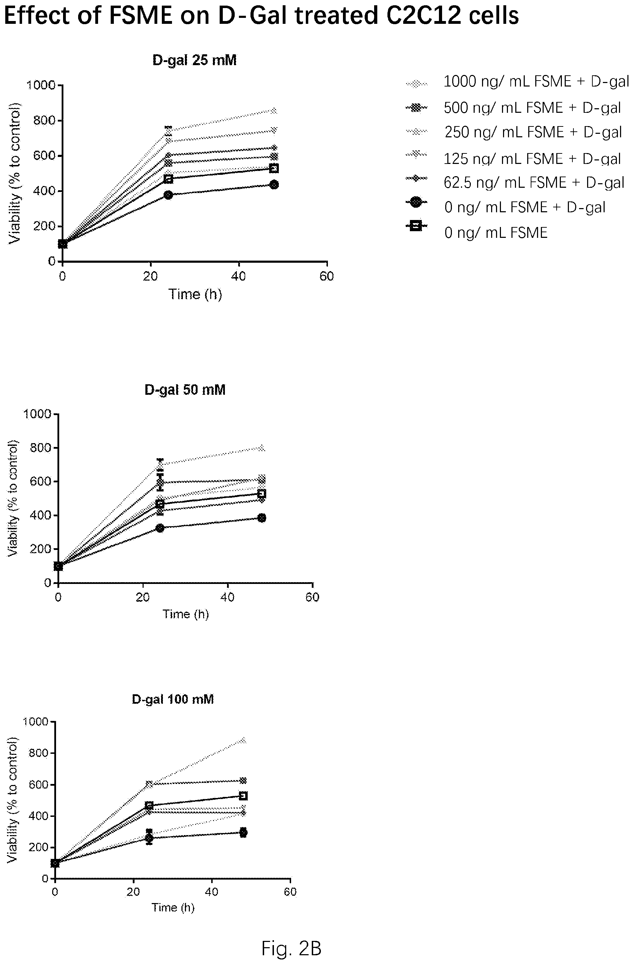

***Results:*** FSME was able to alleviate D-gal induced C2C12 senescence. C2C12 viability is determined by MTT assay. FSME at concentrations between 62.5 ng/ mL - 1000 ng/ mL was able to alleviate C2C12 senescence (**Fig. 2B**).

### EXAMPLE 4: Effect of FSME on HSMM Cell Viability

- **HSMM cells.** Human skeletal muscle myoblasts ("HSMM") were obtained from Lonza (LONZA, CC-2580).
- **Cell culture.** Cells were cultured in SkGM^{™}-2 Skeletal Muscle Cell Growth Medium-2 BulletKit^{™} supplemented with 10% fetal bovine serum (FBS), and incubated at 37 °C in 5% CO₂. Subcultures were performed by using 0.05% trypsin solution when the confluence reached 80%.

### • MTT assay

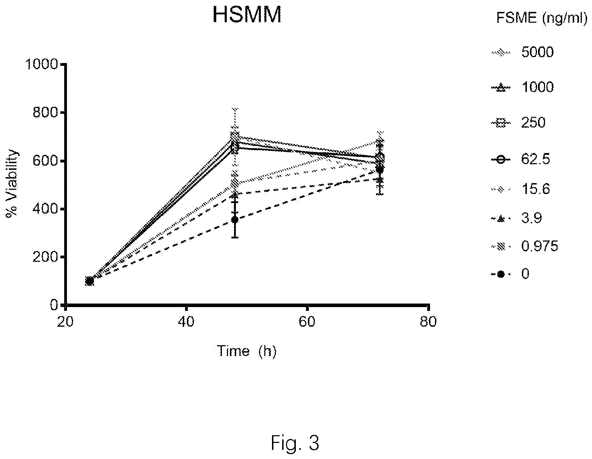

- Cell viability was determined by MTT assay as reflected by the activity of succinate dehydrogenase.
- HSMM (1 × 10⁴ per well) were plated in 96-well microplates. Following incubation with FSME, 3-[4,5-dimethylthiazol-2-yl]-2,5-diphenyl tetrazoliumbromide (MTT, 100 µl, 0.5 mg/ml) was added to each well and the plate was incubated at 37 °C for 3 h. The medium for cell culture was removed and replaced with 100 µl MTT Solubilization Solution, 10% Triton X-100, plus 0.1 N HCl in anhydrous isopropanol to dissolve the formazan crystals. The cell viability was quantified by spectrophotometer by measuring absorbance at 570 nm using a microplate reader. Cells without FSME treatment served as control.
- **Results:** FSME was able to promote HSMM viability and proliferation. As assessed by MTT assay, FSME at concentrations between 0.975 ng/ mL to 1000 ng/ mL were able to promote HSMM viability and proliferation (**Fig. 3**).

### EXAMPLE 5: Effect of FSME on Myogenic Differentiation of C2C12 cells

- **C2C12 cells.** Murine myoblast cell line (ATCC CRL-1772) was obtained from ATCC and used as C2C12 cells.
- **Cell culture.** The C2C12 cells were cultured in Dulbecco modified Eagle medium with high glucose supplemented with 10% (v/v) fetal bovine serum (FBS), and incubated at 37 °C in 5% CO₂. For C2C12 differentiation, 3×10⁴ cells were seeded in 6-well plates and cultured in growth media until reaching 70-80% confluence (day 0). Media were then replaced with Dulbecco modified Eagle medium with high glucose supplemented with 2% (v/v) horse serum. Cells were kept in differentiation medium until the end of the assay, typically between 5 and 7 days.

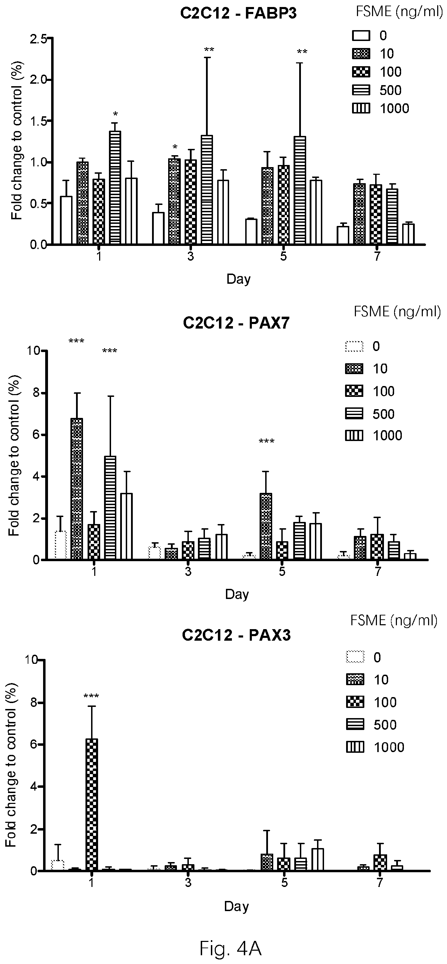

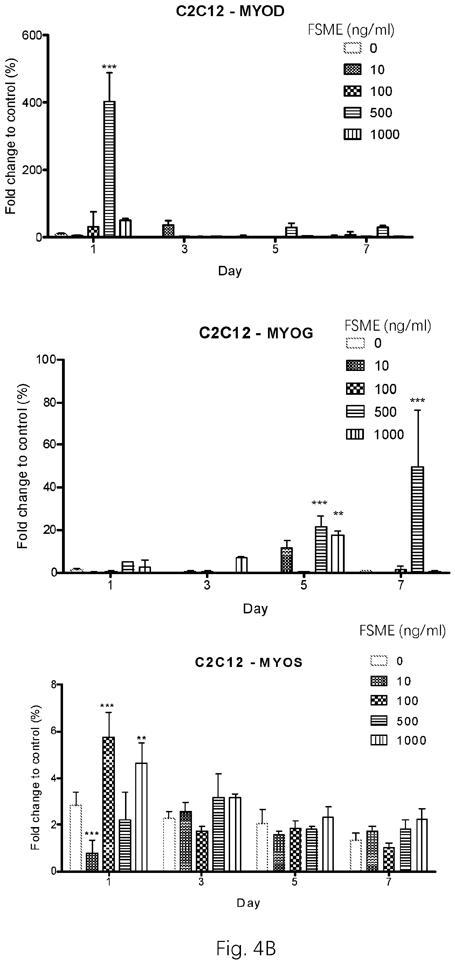

- **Effect of FSME on Myogenic Differentiation of C2C12 cells.** Myotube formation was monitored every two days. The time points for monitoring were 0h, 24h, 3 days ("3D"), 5D and 7D, respectively. Cells were collected for total RNA isolation by Trizol reagent and followed by real time PCR. The expression levels of myogenic precursor markers (Pax3, Pax7 and FABP3) and skeletal muscle markers (MyoD, MyoG and MyoS) were determined with RT-qPCR.
- Results: As shown in **Fig. 4A****,** FSME promoted the expression of myogenic precursor markers, FABP3 and PAX7, in C2C12 cells. FSME at 10 ng/ mL - 1000 ng/ mL was able to significantly upregulate myogenic precursor markers. * represents p< 0.05, ** represents p < 0.01, *** represents p < 0.001. Further, as shown in **Fig. 4B****,** FSME promoted the expression of skeletal muscle markers, MyoD ("MYOD" in Fig. 4B), MyoG ("MYOG" in Fig. 4B) and MyoS ("MYOS" in Fig. 4B), in C2C12 cells. FSME at 10 ng/ mL - 1000 ng/ mL was able to significantly upregulate myogenic precursor markers. ** represents p < 0.01, *** represents p < 0.001.
- These results indicate that FSME is effective in promoting the myogenic differentiation of C2C12 cells.

### EXAMPLE 6: Effect of FSME on Myogenic Differentiation of HSMM cells

- **HSMM cells.** Human skeletal muscle myoblasts ("HSMM") were obtained from Lonza (LONZA, CC-2580).
- **Cell culture.** HSMM cells were cultured in SkGM^{™}-2 Skeletal Muscle Cell Growth Medium-2 BulletKit^{™} supplemented with 10% (v/v) fetal bovine serum (FBS) at 37 °C in 5% CO₂. For HSMM differentiation, 6×10⁴ cells were seeded in 6-well plates and cultured in growth media until reaching 70-80% confluence (Day 0). Media for cell culture were then replaced with Dulbecco modified Eagle medium F-12 supplemented with 2% (v/v) horse serum. Cells were kept in differentiation medium until the end of the assay, typically between 3 and 5 days.

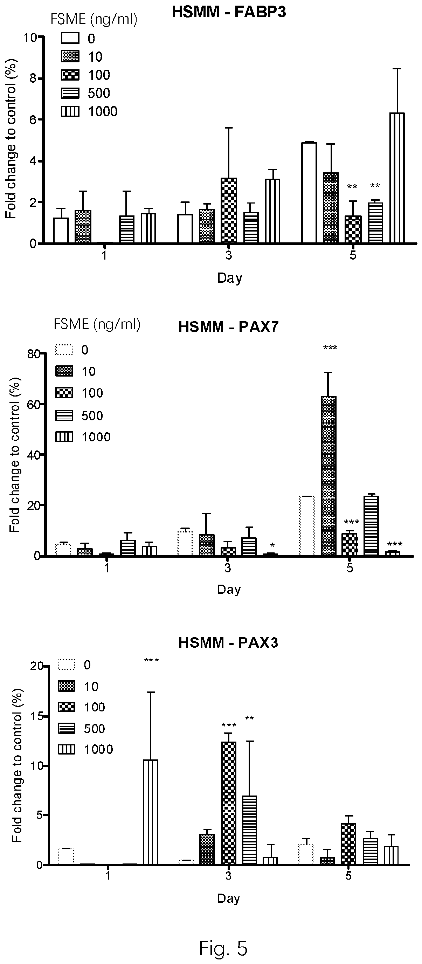

- **Effect of FSME on Myogenic Differentiation of HSMM cells.** Myotube formation was monitored every two days. The time-point are 0h, 24h, 3D and 5D. Cells were collected for total RNA isolation by Trizol reagent and followed by real time PCR. The expression levels of myogenic precursor markers (Pax3, Pax7 and FABP3) were determined with RT-qPCR.
- Results: As shown in **Fig. 5****,** FSME promoted the expression of myogenic precursor markers, FABP3, PAX7 and PAX3, in HSMM cells. FSME at 10 ng/ mL - 1000 ng/ mL was able to significantly upregulate myogenic precursor markers. * represents p< 0.05, ** represents p < 0.01, *** represents p < 0.001.
- These results indicate that FSME is effective in promoting the myogenic differentiation of HSMM cells.

### EXAMPLE 7: Effect of FSME on Muscle Regeneration in C57 Mice

In this experiment, four batches of experiments (batches 001, 002, 003 and 004) were performed. C57 mice were used in this experiment as animal models. The mice were provided by Laboratory Animal Services Centre, CUHK.

Batches 001 and 002 of experiments were performed on mice with muscle injury, particularly "young mice" (three-month-old, male C57/6J mice, also referred to as "young C57 mice" hereinafter) with muscle injury. A muscle injury model was created by intra-muscular injection of barium chloride (BaCl₂), followed by the treatment ("Tx") of FSME via intraperitoneal (FSME IP group) or intramuscular (FSME IM group) injection.

Batches 003 and 004 of experiments were performed on "aged mice" or "old mice" (ten-month-old, male C57/6J mice).

### • 7.1 Batch 001

### • Animal

- Three-month-old, male C57/6J mice (n = 10) were used in this batch of experiment.

### • Chemical injury animal model

- Chemical injury was carried out using i.m. injection of 50 µl of barium chloride (0.12% in sterile PBS) in the tibialis anterior (also referred to as "TA").

### • Treatment of Animals

- All mice were randomized equally into three groups: (1) PBS control group (n = 2); (2) treatment group 1, FSME IM group (n = 4); and (3) treatment group 2, FSME IP group (n = 4). Mice were anesthetized with isoflurane and both treatment groups received single dose injection of FSME (100 µl, 5 mg/ml) at day 2 intraperitoneally (i.p.) or intra-muscularly (i.m.) in the TA, while the vesicle group was injected with 100 µl sterile PBS. No animal became severely ill or died at any time prior to the experimental endpoint.

### • Whole body and Forelimb grip strength test

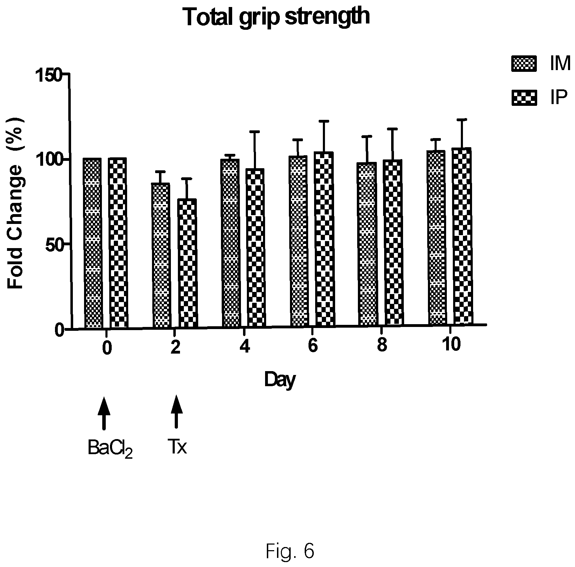

- A grip strength meter was used to measure whole body and forelimb grip strength. As a mouse grasped the grasping-grids, the peak tension in Newton was recorded on a digital force transducer. The gauge was reset to 0 N after stabilization, and the mouse's tail was slowly pulled backward. Tension was recorded by the gauge at the time the mouse released its whole body limbs or forelimbs from the grids. Five measurements per two days were performed.
- Result for grip strength test is shown in **Fig. 6****.** It can be seen that administration of FSME by intraperitoneal or intramuscular route does not cause difference in muscle performance.

### Effect of FSME on Myogenic Differentiation in Young Mice with Muscle Injury

Tibialis anterior and gastrocnemius muscles were isolated from young C57 mice by dissection. The isolated muscle tissue was snap frozen in liquid nitrogen and homogenized in trizol (Invitrogen) and RNA was isolated in accordance to manufacturer's instruction. The isolated RNA samples were reverse transcribed into cDNA using takara reverse transcription kit in accordance to manufacturer's instruction. Real time quantitative PCR (RT-qPCR) was performed using Power SYBR Green PCR kit (Invitrogen) and Applied Biosystem QuantStudio 12K Flexi 384 QPCR system. The expression levels of myogenic precursor markers (Pax3, Pax7 and FABP3) and skeletal muscle markers (MyoD, MyoG and MyoS) were determined with RT-qPCR.

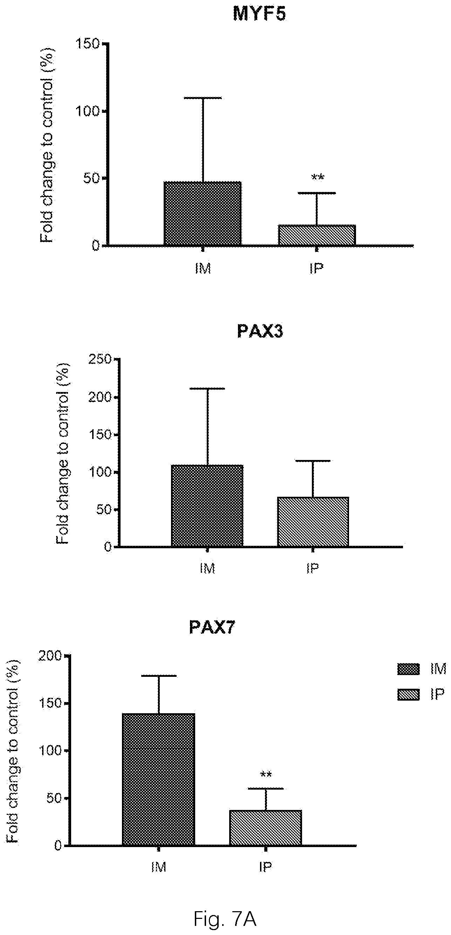

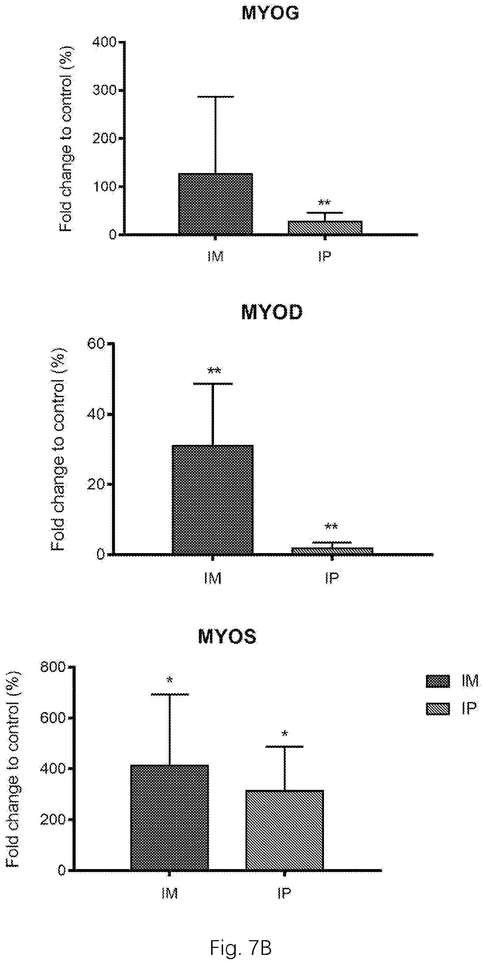

Results are provided in **Fig. 7A** and **7B****.**

As shown in **Fig. 7A****,** single dose FSME administration by intramuscular route significantly increases myogenic precursor markers, Myf5 ("MYF5" in Fig. 7A), Pax3 and Pax7, as compared to intraperitoneal administration in young mice. Gene expression is determined by RT-qPCR. ** represents p < 0.01.

As shown in **Fig. 7B****,** single dose FSME administration by intramuscular route significantly increases skeletal muscle markers, MyoD, MyoG and MyoS, as compared to intraperitoneal administration in young mice. Gene expression is determined by RT-qPCR. * represents p< 0.05, ** represents p < 0.01.

### • 7.2 Batch 002

### • Animal

- Three-month-old, male C57/6J mice (n = 9) were used in this batch of experiment.

### • Chemical injury animal model

- Chemical injury was carried out using i.m. injection of 50 µl of barium chloride (0.12% in sterile PBS) in both TA and gastrocnemius.

### • Treatment of Animals

- All mice were randomized equally into two groups: (1) vesicle group, namely, PBS control group (n = 4); (2) treatment group, namely, FSME IM group (n = 5). Mice were anesthetized with isoflurane and treatment group received bi-weekly injection of FSME (50 µl, 5 mg/ml) at day 2 intra-muscularly (i.m.) in both TA and gastrocnemius, while the vesicle group was replaced with sterile PBS. No animal became severely ill or died at any time prior to the experimental endpoint.

### • Whole body and Forelimb grip strength test

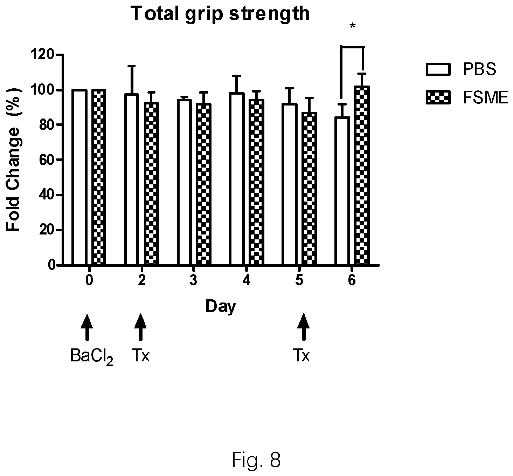

- A grip strength meter was used to measure whole body and forelimb grip strength. As a mouse grasped the grasping-grids, the peak tension in Newton was recorded on a digital force transducer. The gauge was reset to 0 N after stabilization, and the mouse's tail was slowly pulled backward. Tension was recorded by the gauge at the time the mouse released its whole body limbs or forelimbs from the grids. Five measurements per two days were performed.
- Result for grip strength test is shown in **Fig. 8****.** It can be seen that bi-weekly administration of FSME by intramuscular route significantly improve grip strength of young mice post BaCl₂ induced muscular injury. * represents p< 0.05.

### • Light Microscopy Observation

- Young C57 mice gastrocnemius muscle was isolated via dissection. The isolated muscle was embedded in OCT embedding reagent (Invitrogen) and frozen at -80°C until sectioning. The embedded gastrocnemius muscle samples were sectioned (cross section) using a cryotome at a thickness of 10 µm and the sections were mounted onto superfrost slides (Invitrogen). Prior to Hematoxylin and Eosin (H&E) staining, OCT matrix was washed off by immersing slides in PBS for 5 minutes followed by staining with H&E. Samples were first stained with Harris Hematoxylin for 2 minutes and excess hematoxylin was removed by immersing the slides in water and acid alcohol. Hematoxylin stained nuclei were differentiated by immersing sections in Scott's Tap water for 1 minute. Next, the sections were counterstained using 1% eosin solution for 3 minutes and excess eosin was removed by immersing samples in water. Finally, the sections were dehydrated by immersing in increasing concentration of alcohol solution follow by mounting using DPX mounting reagent. Light microscopy analysis was performed using Leica microscope and quantitation of nuclei around the periphery of muscle fiber was performed using Image J analysis.

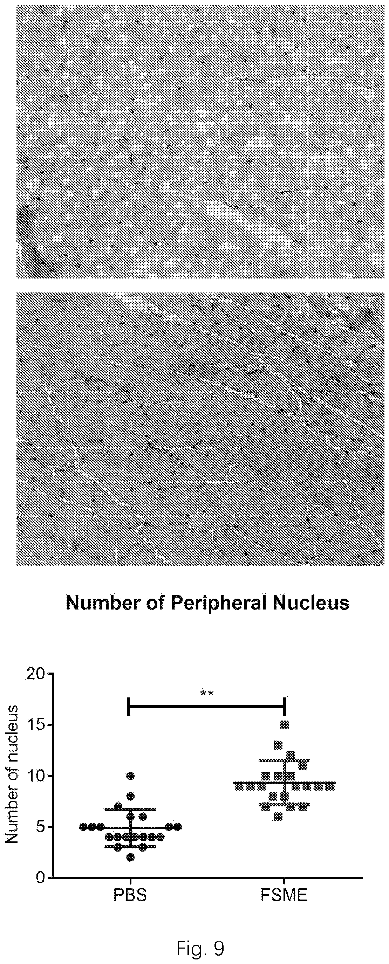

Results: As shown in **Fig. 9****,** bi-weekly administration of FSME by intramuscular route significantly induced muscle repair after BaCl₂ induced muscular injury. BaCl₂ induced injury in muscle fiber while FSME administration induced muscle repair along with cellular infiltration into the muscle periphery. Dot plot represents the average number of nucleus around the muscle periphery in PBS treated and FSME treated muscle. ** represents p <0.01.

### 7.3 Batches 003 and 004

### • Animal

- Ten-month-old, male C57/6J mice (n = 20) which served as "aged mice" or "old mice" were used in this batch of experiment.

### • Treatment of Animals

- All mice were randomized equally into two groups: (1) vehicle group, namely, PBS control group (n = 9); and (2) treatment group, namely, FSME IM group (n = 11). Mice were anesthetized with isoflurane and the treatment group received injection of FSME (100 µl, 5 mg/ml) at day 0 intra-muscularly (i.m.) in gastrocnemius, while the vesicle group was replaced with sterile PBS. Four animals from PBS group and one from treatment group died before the experimental endpoint.

### • Whole body and Forelimb grip strength test

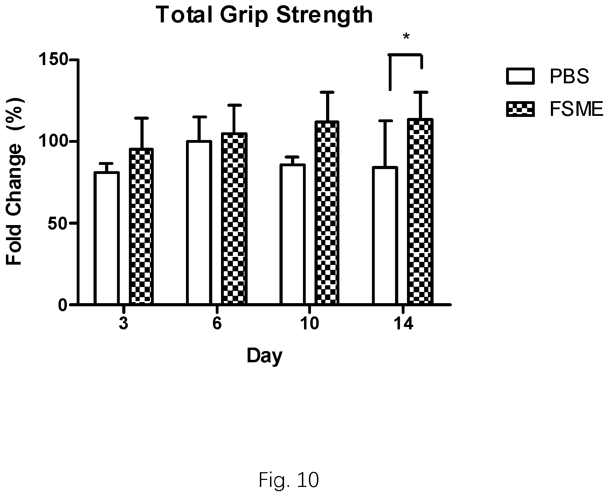

- A grip strength meter was used to measure whole body and forelimb grip strength. As a mouse grasped the grasping-grids, the peak tension in Newton was recorded on a digital force transducer. The gauge was reset to 0 N after stabilization, and the mouse's tail was slowly pulled backward. Tension was recorded by the gauge at the time the mouse released its whole body limbs or forelimbs from the grids. Five measurements per two days were performed.
- Results. As shown in **Fig. 10****,** bi-weekly administration of FSME by intramuscular route for a period of 2 weeks, significantly improved grip strength of old mice. * represents p< 0.05.

### • Effect of FSME on Myogenic Differentiation in Aged Mice

Aged mice gastrocnemius muscle was isolated from old C57 mice by dissection. The isolated muscle tissue was snap frozen in liquid nitrogen and homogenized in trizol (Invitrogen) and RNA was isolated in accordance to manufacturer's instruction. The isolated RNA samples were reverse transcribed into cDNA using takara reverse transcription kit in accordance to manufacturer's instruction. Real time quantitative PCR (RT-qPCR) was performed using Power SYBR Green PCR kit (Invitrogen) and Applied Biosystem QuantStudio 12K Flexi 384 QPCR system.

The expression levels of myogenic precursor markers (Pax3, Myf5 and FABP3) and skeletal muscle markers (MyoD, MyoG and MyoS) were determined with RT-qPCR.

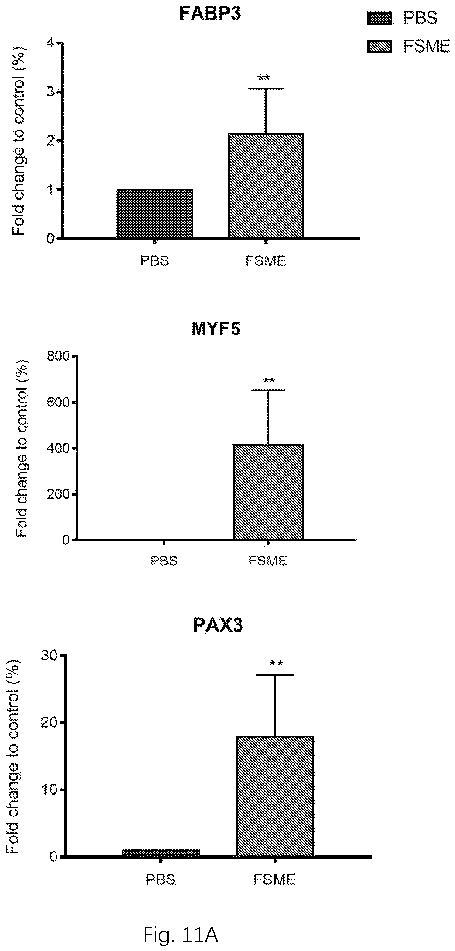

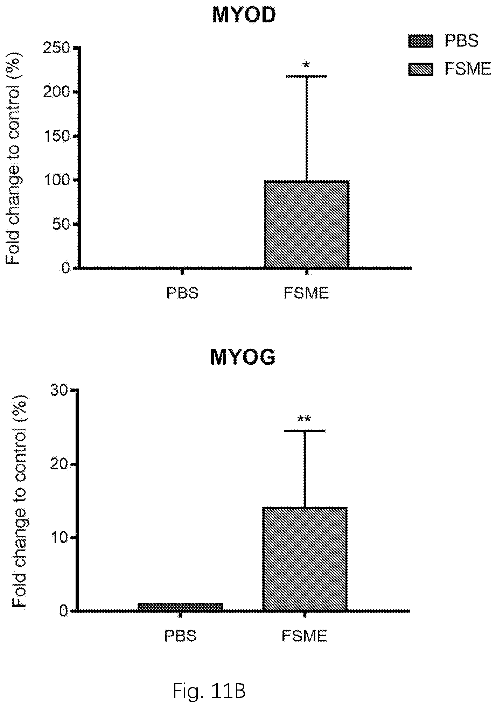

Results are provided in **Fig. 11A** and **11B****.**
- As shown in **Fig. 11A****,** bi-weekly FSME administration by intramuscular route (FSME IM group) significantly increased expression of myogenic precursor markers, Fabp3, Myf5 and Pax3, as compared to the vehicle group in old mice. Gene expression is determined by RT-qPCR. * represents p< 0.05, ** represents p < 0.01.
- As shown in **Fig. 11B****,** bi-weekly FSME administration by intramuscular route (FSME IM group) significantly increased expression of skeletal muscle markers, MyoD and MyoG, as compared to the vehicle group in old mice. Gene expression is determined by RT-qPCR. * represents p< 0.05, ** represents p < 0.01.

From the results above, it can be seen that FSME is effective in the prevention or treatment of muscle disorders or diseases or enhancement of myogenesis.

Specifically, FSME is effective at least in the aspects:
- promoting myoblast proliferation;
- alleviating myoblast senescence induced by D-gal (potential *in vitro* senescence model);
- promoting myogenic differentiation in BaCl₂ induced muscle injury mice; and
- improving muscle strength in both BaCl₂ injured mice and aged mice.

While the present invention has been described with reference to the specific embodiments thereof, it should be understood by those skilled in the art that various changes may be made and equivalents may be substituted without departing from the scope of the invention. In addition, many modifications may be made to adapt a particular situation, material, composition of matter, process, process step or steps, to the objective, and scope of the present invention. All such modifications are intended to be within the scope of the claims appended hereto.

### References:

1. Lexell J, Taylor CC, Sjostrom M. What is the cause of the ageing atrophy? Total number, size and proportion of different fiber types studied in whole vastus lateralis muscle from 15- to 83-year-old men. J Neurol Sci. 1988;84(2-3):275-94.
2. Beaudart C, Zaaria M, Pasleau F, Reginster JY, Bruyere O. Health Outcomes of Sarcopenia: A Systematic Review and Meta-Analysis. PLoS One. 2017;12(1):e0169548.
3. Sayer AA, Dennison EM, Syddall HE, Gilbody HJ, Phillips DI, Cooper C. Type 2 diabetes, muscle strength, and impaired physical function: the tip of the iceberg? Diabetes Care. 2005;28(10):2541-2.
4. Prado CM, Baracos VE, McCargar LJ, Reiman T, Mourtzakis M, Tonkin K, et al. Sarcopenia as a determinant of chemotherapy toxicity and time to tumor progression in metastatic breast cancer patients receiving capecitabine treatment. Clin Cancer Res. 2009;15(8):2920-6.
5. Valter Santilli et al., Clinical definition of sarcopenia. Clinical Cases in Mineral and Bone Metabolism 2014; 11(3): 177-180.

## Claims

1. An extract obtained from one or more tissue(s) of a fetal or newborn animal for use in the prevention or treatment of muscle disorders or diseases in a subject, wherein the tissues are skeletal muscle, and the animal is a sheep, a goat, a pig, a rat, or a mouse, wherein the extract contains no cells, wherein the extract is produced by the steps of:
a. collecting tissues from the fetal or newborn animal;
b. homogenizing the collected tissues to obtain homogenates;
c. optionally, filtrating and/or centrifuging the homogenates to obtain a filtrate; and
d. optionally, purifying/processing the filtrate further to obtain the final products composed of fetal tissue extract.

2. The extract for use according to claim 1, wherein the sheep is a fetal sheep, preferably a 12-18 gestation week fetal sheep.

3. The extract for use according to claim 2, wherein the sheep is free of specific pathogen(s).

4. The extract for use according to claim 1, wherein the extract is fetal sheep muscle extract.

5. The extract for use according to any of claims 1-4, wherein the tissues are collected from the fetal or newborn animal immediately following caesarean section.

6. The extract for use according to any of claims 1-5, wherein the extract is xenogenic or allogenic to the subject.

7. The extract for use according to any of claims 1-6, wherein the muscle disorder or disease is aging-associated muscle disorder or disease, for instance, sarcopenia.

8. The extract for use according to any of claims 1-6, wherein the muscle disorder or disease is muscle injury, for instance, chemical injury.

## Patentansprüche

1. Extrakt, der aus einem oder mehreren Gewebe(n) eines fötalen oder neugeborenen Tieres gewonnen wird, zur Verwendung bei der Vorbeugung oder Behandlung von Muskelstörungen oder -erkrankungen in einem Subjekt, wobei die Gewebe Skelettmuskeln sind und das Tier ein Schaf, eine Ziege, ein Schwein, eine Ratte oder eine Maus ist, wobei der Extrakt keine Zellen enthält, wobei der Extrakt durch die Schritte hergestellt wird:
a. Entnehmen von Geweben aus dem fötalen oder neugeborenen Tier;
b. Homogenisieren der entnommenen Gewebe, um Homogenate zu erhalten;
c. optional Filtrieren und/oder Zentrifugieren der Homogenate, um ein Filtrat zu erhalten; und
d. optional weiteres Reinigen/Verarbeiten des Filtrats, um die Endprodukte zu erhalten, die aus fötalem Gewebeextrakt bestehen.

2. Extrakt zur Verwendung gemäß Anspruch 1, wobei das Schaf ein fötales Schaf, vorzugsweise ein fötales Schaf in der 12.-18. Schwangerschaftswoche, ist.

3. Extrakt zur Verwendung gemäß Anspruch 2, wobei das Schaf frei vom oder von spezifischen Krankheitserreger(n) ist.

4. Extrakt zur Verwendung gemäß Anspruch 1, wobei der Extrakt fötaler Schafsmuskelextrakt ist.

5. Extrakt zur Verwendung gemäß einem der Ansprüche 1-4, wobei die Gewebe aus dem fötalen oder neugeborenen Tier unmittelbar nach einem Kaiserschnitt entnommen sind.

6. Extrakt zur Verwendung gemäß einem der Ansprüche 1-5, wobei der Extrakt für das Subjekt xenogen oder allogen ist.

7. Extrakt zur Verwendung gemäß einem der Ansprüche 1-6, wobei die Muskelstörung oder -erkrankung eine altersbedingte Muskelstörung oder -erkrankung, beispielsweise Sarkopenie, ist.

8. Extrakt zur Verwendung gemäß einem der Ansprüche 1-6, wobei die Muskelstörung oder -erkrankung eine Muskelverletzung, beispielsweise eine chemische Verletzung, ist.

## Revendications

1. Extrait obtenu à partir d'un ou de plusieurs tissu(s) d'un animal foetal ou nouveau-né, destiné à être utilisé dans la prévention ou le traitement de troubles ou maladies musculaires chez un sujet, les tissus étant du muscle squelettique, et l'animal étant un mouton, une chèvre, un porc, un rat ou une souris, l'extrait ne contenant pas de cellules, dans lequel l'extrait est produit par les étapes consistant à :
a. prélever des tissus sur l'animal foetal ou nouveau-né ;
b. homogénéiser les tissus prélevés pour obtenir des homogénats ;
c. en option, filtrer et/ou centrifuger les homogénats pour obtenir un filtrat ; et
d. en option, purifier/traiter davantage le filtrat pour obtenir les produits finals composés d'extrait de tissu foetal.

2. Extrait destiné à être utilisé selon la revendication 1, dans lequel le mouton est un mouton foetal, de préférence un mouton foetal à la 12^{e}-18^{e} semaine de gestation.

3. Extrait destiné à être utilisé selon la revendication 2, dans lequel le mouton est exempt d'agent(s) pathogène(s) particulier(s).

4. Extrait destiné à être utilisé selon la revendication 1, dans lequel l'extrait est un extrait de muscle de mouton foetal.

5. Extrait destiné à être utilisé selon l'une quelconque des revendications 1-4, dans lequel les tissus sont prélevés sur l'animal foetal ou nouveau-né immédiatement après opération césarienne.

6. Extrait destiné à être utilisé selon l'une quelconque des revendications 1-5, dans lequel l'extrait est xénogénique ou allogénique vis-à-vis du sujet.

7. Extrait destiné à être utilisé selon l'une quelconque des revendications 1-6, dans lequel le trouble ou la maladie musculaire est un trouble ou une maladie lié(e) au vieillissement, par exemple, une sarcopénie.

8. Extrait destiné à être utilisé selon l'une quelconque des revendications 1-6, dans lequel le trouble ou la maladie musculaire est une lésion musculaire, par exemple une lésion chimique.
